# EUROPÄISCHE PATENTSCHRIFT

(11) **EP 1 294 389 B1**
(45) Veröffentlichungstag und Bekanntmachung des Hinweises auf die Patenterteilung: **08.03.2006**
(21) Anmeldenummer: 01953844.6
(22) Anmeldetag: 29.06.2001
(51) Int. Cl.: A61K 36/48, A61P 15/12, A61P 19/10, A61P 25/28, A61P 9/00, A61P 13/08

(54) **THERAPEUTISCHE VERWENDUNG VON EXTRAKTEN AUS SOPHORA FLAVESCENS ODER SOPHORA SUBPROSTRATA**
THERAPEUTICAL USE OF SOPHORA FLAVESCENS OR SOPHORA SUBPROSTRATA EXTRACTS
UTILISATION THERAPEUTIQUE D'EXTRAITS DE SOPHORA FLAVESCENS OU DE SOPHORA SUBPROSTRATA

(30) Priorität: 29.06.2000 DE 10031650
(43) Veröffentlichungstag der Anmeldung: 26.03.2003
(73) Patentinhaber: Dr. Willmar Schwabe GmbH & Co. KG, 76227 Karlsruhe (DE)
(72) Erfinder: ERDELMEIER, Clemens, 76139 Karlsruhe (DE); KOCH, Egon, 76229 Karlsruhe (DE)
(74) Vertreter: Adam, Holger
(86) Internationale Anmeldenummer: PCT/DE2001/002461
(87) Internationale Veröffentlichungsnummer: WO 2002/000237

(56) Entgegenhaltungen:
- DATABASE BIOSIS [Online] BIOSCIENCES INFORMATION SERVICE, PHILADELPHIA, PA, US; 1989 XU Q ET AL: "IMMUNOLOGICAL MECHANISMS OF ANTITUMOR ACTIVITY OF SOME KINDS OF CRUDE DRUGS ON TUMOR NECROSIS FACTOR PRODUCTION" Database accession no. PREV199089006631 XP002182663 & INTERNATIONAL JOURNAL OF IMMUNOPHARMACOLOGY, Bd. 11, Nr. 6, 1989, Seiten 607-614, ISSN: 0192-0561
- DATABASE BIOSIS [Online] BIOSCIENCES INFORMATION SERVICE, PHILADELPHIA, PA, US; 1996 MAO HUI-SHENG LIU HONG-YIN ET AL: "Modulation of the malignant phenotype and the immunological response of tumor cells by Sophora flavescens Ait (AF-7)." Database accession no. PREV199799338003 XP002182665 & ZHONGGUO ZHONGLIU LINCHUANG, Bd. 23, Nr. 11, 1996, Seiten 799-803, ISSN: 1000-8179
- OHYAMA M ET AL: "Antitumor Agents 200. Cytotoxicity of Naturally Occurring Resveratrol Oligomers and their Acetate Derivatives" BIOORGANIC & MEDICINAL CHEMISTRY LETTERS, OXFORD, GB, Bd. 9, Nr. 20, 18. Oktober 1999 (1999-10-18), Seiten 3057-3060, XP004180537 ISSN: 0960-894X
- DATABASE BIOSIS [Online] BIOSCIENCES INFORMATION SERVICE, PHILADELPHIA, PA, US; Oktober 2000 (2000-10) KO W G ET AL: "Lavandulylflavonoids: A new class of in vitro apoptogenic agents from Sophora flavescens." Database accession no. PREV200000462363 XP002182664 & TOXICOLOGY IN VITRO, Bd. 14, Nr. 5, Oktober 2000 (2000-10), Seiten 429-433, ISSN: 0887-2333
- PATENT ABSTRACTS OF JAPAN vol. 013, no. 556 (C-664), 11. Dezember 1989 (1989-12-11) & JP 01 230525 A (NITTO DENKO CORP), 14. September 1989 (1989-09-14)

## Beschreibung

Die vorliegende Erfindung betrifft die Verwendung von Extrakten aus *Sophora flavescens* oder *Sophora subprostrata* zur Herstellung eines Medikaments bestehend aus einem Extrakt aus *Sophora flavescens* oder *Sophora subprostrata* und üblichen pharmazeutisch verträglichen Hilfsstoffen zur Prophylaxe und Therapie von Krankheitszuständen, die durch einen Mangel an Östrogenen oder durch eine Dysregulation des Geschlechtshormonstoffwechsels, insbesondere Östrogenstoffwechsels, verursacht werden, wobei
die Krankheitszustände aus der Gruppe bestehend aus Prostatakrebs, Gebärmutterkrebs, klimakterischen Beschwerden, benigne Prostatahyperplasie, Osteoporose, Alzheimersche Krankheit und Herz-Kreislauferkrankungen ausgewählt sind.

Unregelmäßige Menstruationszyklen treten bei Frauen ab einem Alter von etwa 40 Jahren auf und markieren den Beginn der Menopause. Diese Phase von Veränderungen im endokrinen System, Klimakterium genannt, kann eine Dekade und länger anhalten. Sie resultiert aus einer Erschöpfung der Follikelbildung und deren verminderte Reaktion auf Gonadotropin. Als Folge des Follikelausfalls sinkt die Produktion von Östrogen und bleibt schließlich völlig aus. Damit verbunden können sich eine Reihe von Symptomen entwickeln, z.B. Hitzewallungen, Depressionen, Angstzustände, geistige Verwirrtheit, Schlaflosigkeit usw. Außerdem können als Folge des Absinkens der Östrogenproduktion ernsthafte Gesundheitsprobleme wie Osteoporose, Herzinsuffizienz, Schlaganfall und Krebs auftreten.

Obwohl eine Hormonersatztherapie mit Östrogenen zur Erleichterung klimakterischer Beschwerden sehr wirksam ist, gibt es zahlreiche Studien, die zeigen, dass damit ein erhöhtes Risiko für Brust- und Gebärmutterkrebs, Herz-KreislaufErkrankungen und Veränderungen des Lebermetabolismus verbunden sein kann. Durch die Gabe von Progestinen kann das Krebsrisiko vermindert werden. Der protektive Effekt der Progestine scheint jedoch bei einer Langzeitanwendung zu verschwinden und es wird vermutet, dass die Hormonersatztherapie (HRT) mit einem bis zu 35-40 % erhöhten Brustkrebsrisiko verbunden ist. Wegen der Nebenwirkungen und deutlicher Zweifel an der Zuverlässigkeit einer solchen Therapie überhaupt, steht die Medizin der HRT bei Frauen in der Postmenopause eher ablehnend gegenüber. Es wird geschätzt, dass nur ca. 20 % der USamerikanischen Frauen in der Postmenopause eine HRT erhalten, wobei lediglich 40 % dieser Frauen die Therapie über mehr als ein Jahr fortsetzen.

Substanzen, welche bei Mensch oder Tier östrogenagonistisch wirken oder mit Östrogen interagieren, wurden in vielen Pflanzen gefunden. Mindestens 20 verschiedene Stoffgruppen mit östrogenen/antiöstrogenen Eigenschaften aus mehr als 300 Pflanzen von mehr als 16 Pflanzenfamilien wurden bis jetzt gefunden. Die meisten bekannten Phytoöstrogene gehören zu den Gruppen der Isoflavone, Lignane oder Cumestane. Erst kürzlich wurde ein weiteres, hochpotentes Phytoöstrogen identifiziert, das 8-Prenylnaringenin (S.R. Milligan et al., J. Clin. Endocrinol. Metab. 84, 2249-2252 (1999)). Die östrogene Potenz von 8-Prenylnaringenin *in vitro* wurde anhand der Stimulation der alkalischen Phosphatase in Ishikawa-Var-I-Zellen getestet. Dabei fand man, dass 8-Prenylnaringenin wesentlich aktiver war als bislang bekannte Phytoöstrogene wie Coumestrol, Genistein oder Daidzein, und nur wenig schwächer wirkte als 17β-Östradiol.

Epidemiologische und kontrollierte Studien an Populationen in den USA, Europa, China und Japan haben gezeigt, daß ein enge negative Korrelation zwischen der Aufnahme von Phytoöstrogenen mit der Nahrung und dem Auftreten verschiedener Tumoren, insbesondere Brustkrebs und Prostatakarzinom, besteht. Hemmende Wirkungen von Phytoöstrogene auf das Wachstum von Mamma- und Prostatatumorzellen wurde auch in tierexperimentellen Untersuchungen bestätigt. Die präventiven Eigenschaften von Phytoöstrogenen beruhen offenbar auf einer Vielzahl von unterschiedlichen biologischen Eigenschaften, die diese Verbindungen von synthetischen und natürlichen Östrogenen unterscheiden. Abhängig von der Dosis und dem endogenen Hormonstatus verfügen Phytoöstrogene über östrogene oder auch antiöstrogene Wirkungen. Andere biologische Effekte beinhalten z.B. die Hemmung von Tyrosinkinasen, DNA Topoisomerasen, ribosomale S6Kinase, Ornithindecarboxylase, Aromatase oder 5α-Reduktase. Außerdem verfügen Phytoöstrogene häufig über antioxidative Eigenschaften und es ist offensichtlich die Summe dieser unterschiedlichen Aktivitäten, die zur Hemmung der Tumorentstehung und des Tumorwachstums beiträgt (Tham et al., J. Clin. Endocrinol. Metab. 83, 2223-2235 (1998)).

Der vorliegenden Erfindung liegt daher die Aufgabe zugrunde, die Verwendung von Pflanzenextrakten zur Prophylaxe und Therapie von Krankheitszuständen vorzuschlagen, die durch einen Mangel an Östrogenen oder durch eine Dysregulation des Geschlechtshormonstoffwechsels, insbesondere Östrogenstoffwechsels, verursacht werden, wobei
die Krankheitszustände aus der Gruppe bestehend aus Prostatakrebs, Gebärmutterkrebs, klimakterischen Beschwerden, benigne Prostatahyperplasie, Osteoporose, Alzheimersche Krankheit und Herz-Kreislauferkrankungen ausgewählt sind.
Diese Aufgabe wird erfindungsgemäß durch die Verwendung von Extrakten aus *Sophora flavescens* oder *Sophora subprostrata* gemäß dem Patentanspruch 1 gelöst.

Die traditionelle chinesische Medizin kennt einige *Sophora-*Arten (Fabaceae), deren Wurzeln arzneilich verwendet werden. Diese sind *Sophora flavescens, S. subprostrata, S. alopeculoides, S. tonkinensis, S. tomentosa, S. moorcroftiana* und *S. leachiana.* Von diesen kommt *Sophora flavescens* größere Bedeutung zu. Sie ist deshalb Bestandteil des chinesischen Arzneibuchs (Sophorae falvescentis radix, "Ku-shen", Arzneibuch der chinesischen Medizin 9/1997) und wird traditionell zur Behandlung von Durchfall, gastrointestinalen Hämorrhagien und Ekzemen medizinisch verwendet (W. Tang, G. Eisenbrand, Chinese Drugs of Plant Origin, Springer Verlag, Berlin, 1992).

Die Wurzeln von *S. flavescens*, aber auch anderer *Sophora-*Arten enthalten eine Reihe von Chinolizidin-Alkaloiden als Hauptinhaltsstoffe. Das chinesische Arzneibuch schreibt vor, dass der titrimetrische Gesamtgehalt an Alkaloiden 2 % nicht unterschreiten darf. Hauptalkaloide sind Matrin und Oxymatrin.

Neben den Alkaloiden wurde auch eine Vielzahl von Flavonen und verwandten Verbindungen und Triterpensaponine in den Wurzeln von *S. flavescens* sowie etlichen anderen *Sophora*-Arten gefunden.

Verschiedene pharmakologische Eigenschaften sind für *Sophora flavescens* und einzelne Inhaltsstoffe bzw. Inhaltsstoffgruppen beschrieben worden. Insbesondere die Alkaloide werden für antiarrhythmische, antiasthmatische und antitussive Effekte sowie für Antiulcus-Effekte verantwortlich gemacht (Tang und Eisenbrand, s.o.). Auch antineoplastische Effekte und immunsuppressive Eigenschaften wurden beobachtet.

M. Kuroyanagi et al. Journal of Natural Products 1999, 62(12), 1595-1599, berichten über antibakterielle und antiandrogene Flavonoide aus *Sophora flavescens*. Es wurde gefunden, dass nahezu alle aus *Sophora flavescens* isolierten Prenylflavon-Derivate eine antiandrogene Wirkung aufwiesen.

W.M. Mazur et al., Nutritional Biochemistry 1998, 9, 193-200, berichten über die quantitative Bestimmung der Isoflavone Formononetin, Biochanin A, Daidzein, Genistein und Coumestrol und der Lignane Secoisolariciresinol und Mätairesinol in Leguminosensamen, wobei u.a. auch Samen von *Sophora japonica* untersucht worden sind.

*S. flavescens* und *S. japonica* werden in der Toxikologie als toxisch eingestuft. Als Gifte werden vor allem Alkaloide vom Matrin-Typ vermutet (T. Blaszczyk, Deutsche Apoth. Ztg. 2001, 141 (14), 1687-1696). In klinischen Prüfungen wurden auch toxische Effekte wie heftiges Herzklopfen, Atemnot und Krämpfe für die *Sophora*-Alkaloide beobachtet. In Tierversuchen konnten toxische Effekte für Oxymatrin nachgewiesen werden (K.-C. Huang, The Pharmacology of Chinese Herbs, CRC Press, Boca Raton, 1993).

Im Zusammenhang mit der vorliegenden Erfindung werden die *Sophora*-Arten *Sophora flavescens* und *Sophora subprostrata* verwendet für deren Extrakte aus den Wurzeln überraschenderweise im Rahmen der vorliegenden Erfindung starke östrogene Wirkungen festgestellt wurden. Diese Extrakte konnten durch weitere Trennung und Aufreinigung angereichert werden. Es zeigte sich, dass die beobachteten pharmakologischen Effekte durch das Zusammenspiel der in den Extrakten vorliegenden Isoflavone wie Genistein und Daidzein, Flavone wie 8-Prenylnaringenin, Kushenol X, 8-Prenylkämpferol, Leachianon G und Kushenol E, Chalkone und Pterocarpane wie Maackiain und Maackiainglucosid sowie noch nicht identifizierte weitere flavonoidartige Verbindungen verursacht werden. In diesem Zusammenhang ist auch das von den Erfindern neu gefundene Chalkon 2,4,4',6'-Tetrahydroxy-3'-lavandulyl-2'-Methoxychalcon der nachstehenden Formel 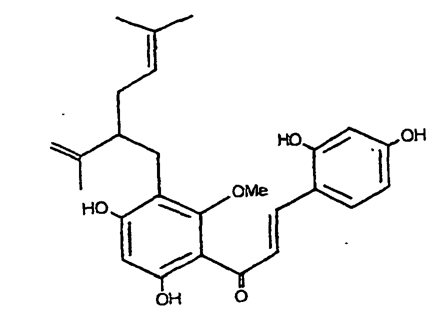 zu nennen.

Figur 1 beschreibt die östrogene Aktivität von 70 % (v/v) (62 % (w/w)) Ethanol-Extrakten (gemäß der Beispiele 1a) und 2a)) aus *Sophora flavescens* und *Sophora subprostrata* in einem Hefe-Assay, verglichen mit 17β-Östradiol.

Figur 2 beschreibt die östrogene Aktivität von Extrakten (70 % (v/v) bzw. 62 % (w/w) Ethanol-Extrakte nach Verteilung mit Ethylacetat; gemäß der Beispiele 1b) und 2b)) aus *Sophora flavescens* und *Sophora subprostrata* in einem Hefe-Assay, verglichen mit 17β-Östradiol.

Figur 3 beschreibt die östrogene Aktivität von Extrakten (60 % (w/w) Ethanol-Extrakte nach Verteilung mit Ethylacetat gemäß Beispiel 3) aus *Sophora flavescens* in einem Hefe-Assay, verglichen mit 17β-Östradiol.

Entsprechende Extrakte können allgemein zum Beispiel durch Extraktion mit einem Lösungsmittel mittlerer Polarität, das aus der Gruppe umfassend wässrige Alkohole, wässrige Ketone und Ester einschließlich wässrigen oder wassergesättigten Estern ausgewählt ist, Abziehen des Lösungsmittels und nachfolgende Flüssig-Flüssig-Verteilung zwischen einem organischen Lösungsmittel und Wasser erhalten werden.

Die vorstehend genannten Alkohole bzw. Ketone können als 10-96 % (v/v) oder (w/w), bzw. 10-99% (v/v) oder (w/w), insbesondere 50-92 % (v/v) oder (w/w) wässrige Gemische verwendet werden, wobei 70 % (v/v) (62 % (w/w)) Ethanol, 60 % (w/w) Ethanol oder wassergesättigtes Ethylacetat ganz besonders bevorzugt sind.

Zur Flüssig-Flüssig-Verteilung können organische Lösungsmittel ausgewählt aus der Gruppe umfassend Ethylacetat, tert-Butylmethylether, n-Butanol und Butanon verwendet werden, wobei Ethylacetat bevorzugt ist.

Die auf diese Weise erhaltenen Extrakte sind dadurch gekennzeichnet, dass der Gesamtalkaloidgehalt unterhalb 0,2 %, vorzugsweise unterhalb 0,1 % liegt. Insbesondere ist der Extrakt alkaloidfrei. Alkaloidfrei bedeutet in diesem Zusammenhang, dass Alkaloidverbindungen mit den gängigen analytischen Verfahren wie HPLC nicht nachweisbar sind.

Erfindungsgemäß werden die Extrakte aus *Sophora flavescens* und *Sophora subprostrata* zur Herstellung eines Medikaments bestehend aus einem Extrakt aus *Sophora flavescens* oder *Sophora subprostrata* und üblichen pharmazeutisch verträglichen Hilfsstoffen zur Prophylaxe und Therapie von Krankheitszuständen verwendet, die durch einen Mangel an Östrogenen oder durch eine Dysregulation des Geschlechtshormonstoffwechsels, insbesondere Östrogenstoffwechsels, verursacht werden, wobei
die Krankheitszustände aus der Gruppe bestehend aus Prostatakrebs, Gebärmutterkrebs, klimakterischen Beschwerden, benigne Prostatahyperplasie, Osteoporose, Alzheimersche Krankheit und Herz-Kreislauferkrankungen ausgewählt sind.

Die erhaltenen Extrakte können zusammen mit üblichen pharmazeutisch verträglichen Hilfsstoffen zu pharmazeutischen Zubereitungen wie Kapseln, Filmtabletten und Dragees verarbeitet werden, wobei als übliche pharmazeutisch verträgliche Hilfsstoffe Füll-, Binde-, Spreng-, Schmier- und Überzugsmittel für Filmtabletten und Dragees sowie Öle und Fette als Füllmassen für Kapseln verwendet werden können.

Die Extrakte werden am Menschen in einer Dosierung von 1-1000 mg/täglich, vorzugsweise 100-1000 mg/täglich angewandt.

Die nachstehend angegebenen Beispiele erläutern die Erfindung und sind nicht beschränkend aufzufassen. Alle Prozentangaben beziehen sich auf das Gewicht, falls nichts Anderes angegeben ist.

### Beispiel 1: Herstellung eines Extraktes aus Sophora flavescens

### a) Extraktion

2 kg gemahlene Wurzeln von *S. flavescens* wurden mit 14 kg 70 % (v/v) (62 % (w/w)) Ethanol versetzt, 5 min wirbelextrahiert (Ultraturrax) und 1 h bei 50°C intensiv gerührt. Anschließend wurde über ein Supra Seitz 1500-Filter abgesaugt und der Drogenrückstand in der gleichen Weise noch ein zweites Mal extrahiert. Die beiden Extraktlösungen wurden vereinigt und mit einem Aliquot wurde der Trockenextraktgehalt bestimmt. Es ergab sich ein Trockenrückstand von 475,6 g entsprechend einer Ausbeute von 23,8 %.

### b) Verteilung

Der Extraktionslösung wurde am Rotationsverdampfer bei 50°C das Ethanol entzogen. Der wässrige Rückstand (5 1) wurde 3x mit jeweils 2 1 Ethylacetat ausgerührt, die Ethylacetat-Phasen vereinigt und am Rotationsverdampfer zur Trockne eingeengt:
Ethylacetat-Extrakt = 67,84 g (3,4 % bez. auf Droge; 14,3 % bez. auf Gesamtextrakt)

Der Ethylacetat-Extrakt enthält gemäß HPLC-Analyse Flavone, Isoflavone, Chalkone und Pterocarpane (z.B. 8-Prenylnaringenin, Daidzein, Kushenol X, Norkurarinon, Maackiain, Genistein, 8-Prenylkämpferol). Alkaloide konnten nicht nachgewiesen werden.

Diese Substanzkombination ist in idealer Weise zur Prophylaxe und Therapie von Krankheitszuständen geeignet, die durch einen Mangel an Östrogenen oder durch eine Dysregulation des Geschlechtshormonstoffwechsels, insbesondere Östrogenstoffwechsels verursacht werden (vgl. Beispiel 5).

### Beispiel 2: Herstellung eines Extraktes aus Sophora subprostrata

### a) Extraktion

1 kg gemahlene Wurzeln von *S. subprostrata* wurden mit 7 kg 70 % (v/v) (62 % (w/w)) Ethanol versetzt, 5 min wirbelextrahiert (Ultraturrax) und 1 h bei 50°C intensiv gerührt. Anschließend wurde über ein Supra Seitz 1500-Filter abgesaugt und der Drogenrückstand in der gleichen Weise noch ein zweites Mal extrahiert. Die beiden Extraktlösungen wurden vereinigt und mit einem Aliquot wurde der Trockenextraktgehalt bestimmt. Es ergab sich ein Trockenrückstand von 111,8 g entsprechend einer Ausbeute von 11,2 %.

### b) Verteilung

Der Extraktionslösung wurde am Rotationsverdampfer bei 50°C das Ethanol entzogen. Der wässrige Rückstand (2,5 l) wurde 3x mit jeweils 1 l Ethylacetat ausgerührt, die Ethylacetat-Phasen vereinigt und am Rotationsverdampfer zur Trockne eingeengt:
Ethylacetat-Extrakt = 10,4 g (1,0 % bez. auf Droge; 9,3 % bez. auf Gesamtextrakt)

### Beispiel 3

36 kg gemahlene *Sophora flavescens* Droge wurden mit der 7,7-fachen Gewichtsmenge 60 Gew.% Ethanol versetzt. Unter starkem Rühren und über Dispax (Ultraturrax) im Kreislauf wurde die Extraktlösung 1 h bei 50°C extrahiert. Die Extraktlösung wurde klarfiltriert, der Drogenrückstand noch einmal mit der 7-fachen Gewichtsmenge 60Gew.% Ethanol unter gleichen Bedingungen extrahiert und anschließend filtriert.

Die Extraktlösung wurde dann im Zentrifugalverdampfer auf einen Ethanol Gehalt von 3,5% und einen Trockenextraktgehalt von 10,52% eingeengt. Anschließend wurde diese konzentrierte Lösung 3 mal mit 1:0,4 Volumenteilen gegen wassergesättigten Ethylacetat verteilt. Die Ethylacetat-Phase wurde am Rotationsvedampfer eingeengt und im Trockenschrank bei 60°C nachgetrocknet.

Es resultierten:
Ethylacetat-Extrakt: 1,188 kg nach Vermahlung (3,3% bez. auf Droge)

### HPLC-Gehalte pharmakologisch relevanter Inhaltsstoffe:

| | |
|---|---|
| Maackiainglucosid | 2,8 % |
| 8-Prenylnaringenin | 0,4 % |
| Maackiain | 0,6 % |
| Kushenol X | 1,3 % |
| 8-Prenylkämpferol | 0,7 % |
| Leachianon G | 6,6 % |
| Kushenol E | 0,3 %. |
| 2,4,4',6'-Tetrahydroxy-3'-lavandulyl-2'-Methoxychalcon | 0,7 % |

Daidzein und Genistein konnten ebenfalls nachgewiesen werden. Alkaloide waren nicht nachweisbar.

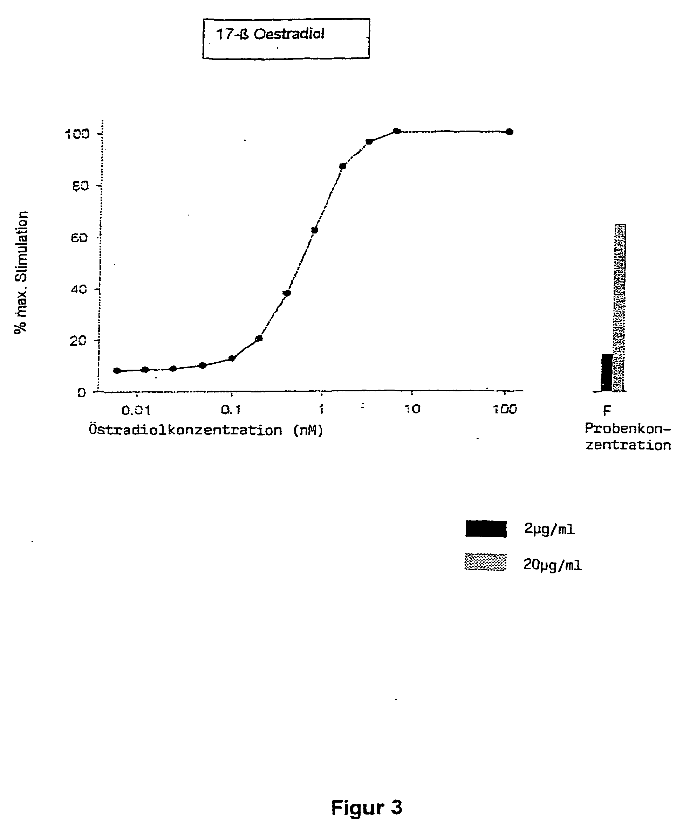

Die östrogene Aktivität konnte für jeden der angegebenen Inhaltsstoffe in einem Hefe-Assay entsprechend Beispiel 5, nachgewiesen werden. Für den Extrakt ergibt sich die östrogene Aktivität aus dem Reportergen-Assay unter Verwendung von Hefezellen gemäß Beispiel 5; vgl. Figur 3.

### HPLC-Methode

| **Säule** | **LiChrospher 100 5 µm, 250 x 4 mm** | |
|---|---|---|
| Eluens | A: 1000 ml bidest Wasser / 3 ml Phosphorsäure (85%)/ 2 ml Triethylamin | |
| | B:100 ml Acetonitril/ 3 ml Phosphorsäure (85%)/ 2 ml Triethylamin /60 ml bidest Wasser | |
| Gradient | % A | % B |
| | 0 min 70 | 30 |
| | 30 min 30 | 70 |
| | 35 min 0 | 100 |
| Fluß | 1,2 ml/min | |
| Detektion | 220 nm | |

### Beispiel 4

150 g gemahlene *Sophora flavescens* Droge wurde zweimal mit wassergesättigtem Ethylacetat im Verhältnis 1:7 (m/m) extrahiert. Dazu wurde die Droge zuvor jeweils 5 Minuten mit einem Ultra-Turrax aufgeschlossen (Bewegungsextraktion). Die Droge wurde sodann je 1 h bei 60°C unter Rückfluss extrahiert. Die vereinigten Extraktlösungen wurden danach abfiltriert und die erhaltene Ethylacetat-Extraktlösung zweimal mit Ethylacetatgesättigtem Wasser im Verhältnis 1:1 (V/V) zurückgeschüttelt. Die vereinigten Ethylacetat-Phasen wurden zur Trockene eingeengt: ,
Ausbeute: 3,99% bez. auf die Droge

### Beispiel 5: Prüfung der hergestellten Ethylacetatextrakte auf östrogene Aktivität mit transfizierten Hefezellen, die den menschlichen α-Östrogenrezeptor exprimieren.

Die Prüfung von Extrakten auf östrogene Eigenschaften erfolgte mit einem Reportergen-Assay unter Verwendung von Hefezellen (Saccharomyces). Die Zellen sind stabil mit dem humanen α-Östrogenrezeptor und einem Expressionsplasmid, das ein Östrogenresponse-Element und das Gen für das Enzym β-Galaktosidase enthält, transfiziert. Alle Proben wurden in einer Konzentration von 20 mg/ml in DMSO gelöst und unverdünnt oder nach Verdünnen mit DMSO im Verhältnis 1/10, 1/100 oder in einem Volumen von 1 µl zu 100 µl Kulturmedium in 96-Well Flachboden-Mikrotiterplatten gegeben. Anschließend wurden 100 µl Hefesuspension und das chromogene Substrat Chlorphenolrot-β-D-Galactopyranosid zugefügt. Auf jeder Platte wurden zur Kontrolle Wells vorbereitet, in die nur Kulturmedium bzw. das Lösungsmittel eingefüllt würde oder die die Standardkonzentrationen von 17β-Östradiol enthielten. Die Hefezellen wurden 72 h bei 32°C inkubiert und dann wurde die Absorption des Mediums bei 540 nm in einem Mikrotiterplatten-Photometer gemessen. Die Proben wurden teilweise zweimal geprüft.

### Ergebnisse:

### Probenkonzentrationen:

| **Verdünnung** | **Konzentration** |
|---|---|
| unverdünnt | 100 µg/ml |
| 1:10 | 10 µg/ml |
| 1:100 | 1 µg/ml |

### Probenbezeichnung:

| **Proben-Nr.** | **Probe** | **Versuch** | ***Sophora*-Art** |
|---|---|---|---|
| A | 70 % (v/v) (62 % (w/w)) Ethanol-Extrakt gemäß Beispiel 1a) | 1 | *Sophora flavescens* |
| B | 70 % (v/v) (62 % (w/w)) Ethanol-Extrakt gemäß Beispiel 2a) | 1 | *Sophora subprostrata* |
| C | 70 % (v/v) (62 % (w/w)) Ethanol-Extrakt gemäß Beispiel 1a) | 2 | *Sophora flavescens* |
| D | Ethylacetat-Extrakt gemäß Beispiel 1b) | 1 | *Sophora flavescens* |
| E | Ethylacetat-Extrakt gemäß Beispiel 2b) | 1 | *Sophora subprostrata* |
| F | Ethylacetat-Extrakt gemäß Beispiel 3 | 1 | *Sophora flavescens* |

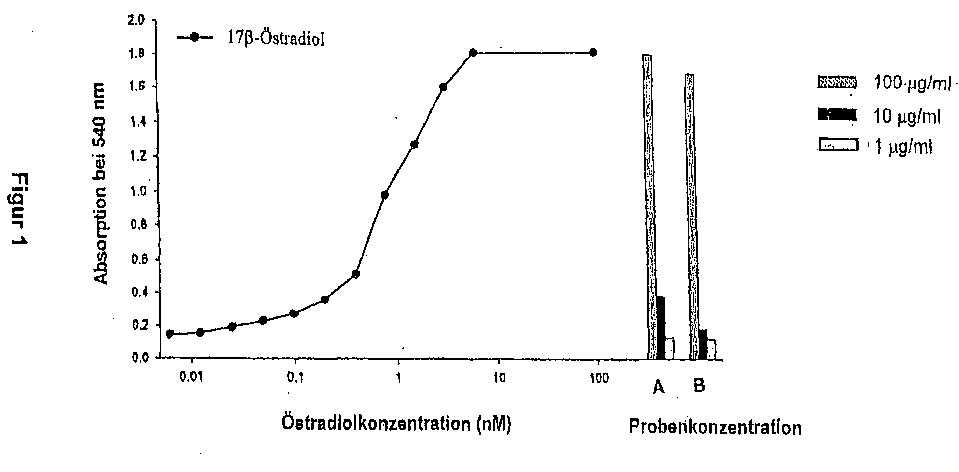

Die Ergebnisse des Assays sind in den Fig. 1, 2 und 3 dargestellt. Hierbei wird die östrogene Aktivität der Extrakte mit der Aktivität von 17β-Östradiol verglichen. Als "aktiv" können hierbei jene Extrakte bezeichnet werden, deren Aktivität beim Vergleich mit 17β-Östradiol einer 17β-Östradiol-Aktivität entspricht, die innerhalb des deutlich ansteigenden Abschnitts (also ab einer Konzentration von etwa 0,1-0,2 nM 17β-Östradiol) der Aktivitätskurve des 17β-Östradiol angesiedelt ist.

Insbesondere beschreibt Figur 1 die östrogene Aktivität von 70 % (v/v) (62 % (w/w)) Ethanol-Extrakten (gemäß der Beispiele 1a) und 2a)) aus *Sophora flavescens* und *Sophora subprostrata* in einem Hefe-Assay, verglichen mit 17β-Östradiol.

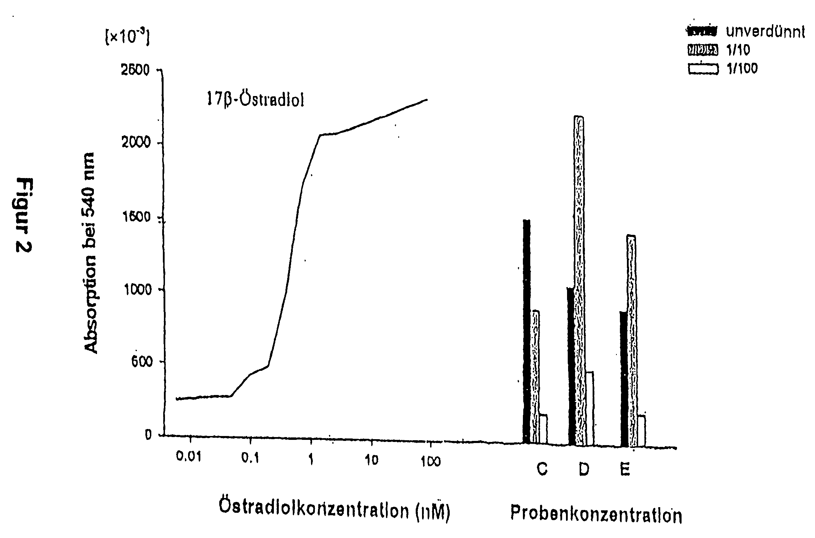

Figur 2 beschreibt die östrogene Aktivität von Extrakten (70 % (v/v) bzw. 62 % (w/w) Ethanol-Extrakte nach Verteilung mit Ethylacetat; gemäß der Beispiele 1b) und 2b)) aus *Sophora flavescens* und *Sophora subprostrata* in einem Hefe-Assay, verglichen mit 17β-Östradiol.

Figur 3 beschreibt die östrogene Aktivität von Extrakten (60 % (w/w) Ethanol-Extrakte nach Verteilung mit Ethylacetat gemäß Beispiel 3) aus *Sophora flavescens* in einem Hefe-Assay, verglichen mit 17β-Östradiol.

## Patentansprüche

1. Verwendung eines Extraktes aus *Sophora flavescens* oder *Sophora subprostrata* zur Herstellung eines Medikaments bestehend aus einem Extrakt aus *Sophora flavescens* oder *Sophora subprostrata* und üblichen pharmazeutisch verträglichen Hilfsstoffen zur Prophylaxe und Therapie von Krankheitszuständen, die durch einen Mangel an Östrogenen oder durch eine Dysregulation des Geschlechtshormonstoffwechsels, insbesondere Östrogenstoffwechsels, verursacht werden, wobei die Krankheitszustände aus der Gruppe bestehend aus Prostatakrebs, Gebärmutterkrebs, klimakterischen Beschwerden, benigne Prostatahyperplasie, Osteoporose, Alzheimersche Krankheit und Herz-Kreislauferkrankungen ausgewählt sind.

## Claims

1. Use of an extract of *Sophora flavescens* or *Sophora subprostrata* for the preparation of a medicament consisting of an extract of *Sophora flavescens* or *Sophora subprostrata* and conventional pharmaceutically-acceptable additives for the prophylaxis and treatment of pathological conditions caused by a deficiency of oestrogens or by a dysregulation of sex hormone metabolism, particularly oestrogen metabolism, wherein the pathological conditions are selected from the group consisting of prostate cancer, cancer of the womb, climacteric complaints, benign prostate hyperplasia, osteoporosis, Alzheimer's disease and cardiovascular diseases.

## Revendications

1. Utilisation d'un extrait de *sophora flavescens* ou de *sophora subprostata* pour fabriquer un médicament constitué d'un extrait de *sophora flavescens* ou de *sophora subprostata* et d'adjuvants habituels qui sont pharmaceutiquement compatibles en vue de la prophylaxie et de la thérapie d'états maladifs, qui sont provoqués par un manque d'oestrogènes ou par une dysrégulation du métabolisme hormonal sexuel, notamment du métabolisme oestrogénique, les états maladifs étant sélectionnés parmi le groupe constitué du cancer de la prostate, du cancer de l'utérus, des troubles climactériques, de l'hyperplasie bénigne de la prostate, de l'ostéoporose, de la maladie d'Alzheimer et des maladies cardio-vasculaires.
